# EUROPÄISCHE PATENTSCHRIFT

(11) **EP 1 651 959 B1**
(45) Veröffentlichungstag und Bekanntmachung des Hinweises auf die Patenterteilung: **12.03.2008**
(21) Anmeldenummer: 04763822.6
(22) Anmeldetag: 05.08.2004
(51) Int. Cl.: G01N 33/542

(54) **MOLEKULARE SONDE UND MATERIAL ZUM NACHWEIS EINES ANALYTS UND DEREN VERWENDUNG**
MOLECULAR PROBE AND MATERIAL FOR THE DETECTION OF AN ANALYTE, AND USE THEREOF
SONDE MOLECULAIRE ET MATERIAU DE DETECTION D'UN ANALYTE ET LEUR UTILISATION

(30) Priorität: 08.08.2003 DE 10337668
(43) Veröffentlichungstag der Anmeldung: 03.05.2006
(73) Patentinhaber: Wessig, Pablo, 13353 Berlin (DE)
(72) Erfinder: WESSIG, Pablo, 13353 Berlin (DE); SCHEDLER, Uwe, 10119 Berlin (DE)
(74) Vertreter: Reinstädler, Diane
(86) Internationale Anmeldenummer: PCT/EP2004/008781
(87) Internationale Veröffentlichungsnummer: WO 2005/016398

(56) Entgegenhaltungen:
- US-A1- 2002 165 364
- US-B1- 6 586 256
- RAKER JOSEPH ET AL: "Selectivity via cooperative interactions: Detection of dicarboxylates in water by a pinwheel chemosensor" JOURNAL OF ORGANIC CHEMISTRY, Bd. 67, Nr. 17, 23. August 2002 (2002-08-23), Seiten 6113-6116, XP002313522 ISSN: 0022-3263
- PRASANNA DE SILVA A ET AL: "SIGNALING RECOGNITION EVENTS WITH FLUORESCENT SENSORS AND SWITCHES" CHEMICAL REVIEWS, AMERICAN CHEMICAL SOCIETY. EASTON, US, Bd. 97, 1997, Seiten 1515-1566, XP000861692 ISSN: 0009-2665
- BROUDE N E: "Stem-loop oligonucleotides: a robust tool for molecular biology and biotechnology" TRENDS IN BIOTECHNOLOGY, ELSEVIER PUBLICATIONS, CAMBRIDGE, GB, Bd. 20, Nr. 6, 1. Juni 2002 (2002-06-01), Seiten 249-256, XP004352763 ISSN: 0167-7799
- ROMOSER V A ET AL: "DETECTION IN LIVING CELLS OF CA2+ -DEPENDENT CHANGES IN THE FLUORESCENCE EMISSION OF AN INDICATOR COMPOSED OF TWO GREEN FLUORESCENT PROTEIN VARIANTS LINKED BY A CLAMODULIN-BINDING SEQUENCE" JOURNAL OF BIOLOGICAL CHEMISTRY, AMERICAN SOCIETY OF BIOLOGICAL CHEMISTS, BALTIMORE, MD, US, Bd. 272, Nr. 20, 16. Mai 1997 (1997-05-16), Seiten 13270-13274, XP002058387 ISSN: 0021-9258

## Beschreibung

Die Erfindung betrifft eine molekulare Sonde und ein Material jeweils zum Nachweis eines Analyts sowie eine Verwendung der molekularen Sonde beziehungsweise des Materials.

Der spezifische Nachweis von Analyten, umfassend Ionen jeglicher Größe als auch nieder- bis hochmolekulare Verbindungen bis hin zu Biopolymeren spielt eine wesentliche Rolle in der chemischen, biochemischen und klinischen Analytik. Dabei kann der Nachweis sowohl die qualitative als auch die quantitative Bestimmung des Analyts einschließen. Neben der Verwendung natürlicher Rezeptoren des betreffenden Analyts, beispielsweise Antikörper zum Nachweis eines Antigens oder Oligonukleotide zum Nachweis von komplementären Nukleinsäuren, ist bekannt, artifizielle Rezeptoren, so genannte molekulare Sonden einzusetzen.

Die Funktionsweise einer molekularen Sonde basiert prinzipiell auf einem Zusammenspiel von drei Funktionen, nämlich der Detektion des Analyts, der Signaltransduktion sowie der Signalgenerierung. Die Detektion umfasst die spezifische und selektive Bindung des Analyts durch die Sonde. Die Umsetzung des Detektionsereignisses in ein auswertbares Signal wird durch die Signaltransduktion gewährleistet. Schließlich muss das Signal an die Umgebung abgegeben werden, wofür die Signalgenerierung zuständig ist. Diese drei Funktionalitäten können in einer konkreten Sonde räumlich und funktional separiert oder auch ineinander greifend vorliegen.

Für das Detektionsereignis wird in der Regel eine Reihe schwacher, nichtbindender Wechselwirkungen zwischen dem Analyt und der Detektorfunktion ausgenutzt. Den Protein-Protein- und Protein-Ligand-Wechselwirkungen in biologischen Systemen nachempfunden ist beispielsweise die Bindung auf der Grundlage von Wasserstoffbrückenbindungen (z.B. US 6,025,505 PS). Nach diesem Prinzip kann eine Reihe biologisch relevanter Moleküle, beispielsweise Creatinin (D. L. Beckles et al., Tetrahedron 51, 1991, 363), Nukleotide (T. W. Bell et al., Angew. Chem. 107, 1995, 2321), Phosphationen (P. Schiessl, F. P. Schmidtchen, J. Org. Chem. 59, 1994, 509) oder Kohlenhydrate (M. Mazik, W. Sicking, Chem. Eur. J. 7, 2001, 664) detektiert werden. Auch die hochspezifischen Wechselwirkungen von Peptiden mit Antikörpern und die Hybridisierung von Oligonukleotiden ist, zumindest teilweise, diesem Prinzip zuzurechnen.

Der Nachweis bestimmter Metallkationen basiert in der Regel auf spezifischen Metall-Ligand-Wechselwirkungen, die durch Art und räumliche Anordnung der die Detektionsfunktion übernehmenden Liganden bestimmt werden. Hierfür existiert eine Vielzahl von Anwendungen, die in Übersichtsartikeln zusammengefasst wurden (z. B. B. Valeur et al., "Cation-Responsive Fluorescent Sensors" in J. P. Desvergne; A.W. Czarnik (Hrsg.); "Chemosensors of Ion and Molecule Recognition", NATO ASI Series, Series C: Mathematical and Physical Sciences - Vol. 492, Kluwer, 1997).

Supramolekulare Host-Guest-Wechselwirkungen, die auf der spezifischen Ausfüllung molekularer Hohlräume durch bestimmte Analyte basieren, wurden zum Nachweis kleiner organischer Moleküle benutzt. Besonders bewährt haben sich hier Cyclodextrine, die über einen konischen, hydrophoben Hohlraum verfügen (Ueno et al., J. Chem. Soc. Chem. Commun. 1988, 1373; J. Am. Chem. Soc. 1989, 111, 6391; Anal. Chem. 1990, 62, 2461).

Die hochspezifische Detektion bestimmter Kohlenhydrate - eine Problemstellung mit großer medizinisch diagnostischer Bedeutung - erfolgt etwa durch Bindung an aromatische Boronsäuren (S. Arimori, M. Takeuchi, S. Shinkai, Chem. Lett. 1996, 77; M. Takeuchi, T. Imada, S. Shinkai, J. Am. Chem. Soc. 118, 1996, 10658).

Im Gegensatz zum vorstehend beschriebenen Detektionsereignis lässt sich die Realisierung der Signaltransduktion weniger klar gliedern. Sehr häufig erfolgen konformative Änderungen des Sondenmoleküls, die dann die Signalgenerierung auslösen. Hier seien exemplarisch die durch Disaccharide ausgelösten Konformationsänderungen in Stilben-diboronsäuren (Sandanayake et al., J. Chem. Soc. 1994, 1621) und Polymethin-diboronsäuren (Takeuchi et al., Tetrahedron 1996, 52, 1195) genannt. Auch die Änderung elektronischer Eigenschaften funktioneller Gruppen durch Bindung des Analyts, zum Beispiel von Aminogruppen bei Komplexierung mit Metallkationen (de Silva et al., J. Chem. Soc. Chem. Commu. 1986, 1709) wird häufig ausgenutzt.

Bei der Signalgenerierung wird ein in der Regel spektroskopisch erfassbares Signal erzeugt, wobei abhängig von der Natur des Signals verschiedene spektroskopische Methoden eingesetzt werden, beispielsweise NMR-, UV-, ESR- oder Fluoreszenz-Spektroskopie. Dabei kommt der Auswertung von Fluoreszenzsignalen heute aufgrund der im Vergleich zu den anderen Methoden wesentlich höheren Nachweisempfindlichkeit mit Abstand die größte Bedeutung zu. Dem Zustandekommen eines Fluoreszenzsignals liegen verschiedene Prinzipien zugrunde. Besonders häufig, insbesondere bei biochemischen Anwendungen, findet der so genannte Fluoreszenz-Resonanzenergie-Transfer (FRET) Verwendung (Selvin, Methods of Enzymology 1995, 246, 300; Selvin, Nat. Struct. Biol. 2000, 7, 730; US 2003/0096242 A). Weitere häufig verwendete Prinzipien zur Signalgenerierung basieren auf der abstandsabhängigen Bildung von Excimeren und deren Fluoreszenz (Zachariasse et al., Biophys. Biochim. Acta 1982, 688, 323; Kakizawa et al., Chem. Lett. 1993, 1671; Aoki et al., J. Chem. Soc. Chem. Comm. 1992, 1341) oder auf der abstandsabhängigen Löschung der Fluoreszenz, zum Beispiel durch Elektronentransfer (Neuweiler et al., Angew. Chem. 2002, 114, 4964; WO 03/040680 A).

Praktisch allen bisher beschriebenen Sondensystemen ist gemeinsam, dass sie für ein spezielles Nachweisproblem, das heißt ein bestimmtes Analyt, und auch für eine bestimmte Signalgenerierung entwickelt wurden. Dies bringt mit sich, dass eine einfache Anpassung der Sonden an andere Problemstellungen in der Regel nicht oder nur sehr schwer möglich ist.

US 6,586,256 B1 beschreibt eine molekulare Sonde zum Nachweis eines Analyts, welche eine Grundstruktur mit einer molekularen Achse umfasst, die eine oder zwei CC-Dreifachbindungen enthält, sowie jeweils eine Triphenylmethylgruppe an jedem Achsenende. Die Phenylreste sind mit insgesamt vier Detektorgruppen sowie mit zwei Signalgruppen (Fluorophore) substituiert. Die Sonde weist eine signalgebende Konformation unter Bindung des Analyts auf sowie eine stumme Konformation in seiner Abwesenheit. Ähnliche Sonden sind aus Raker, J. & Timothy, E.G. (J. Org. Chem. 67, 2002, 6113-6116) bekannt, in denen jedoch die molekulare Achse durch eine 1,4-Diprop-1-in-Phenolgruppe ersetzt ist. Derartige Strukturen sind in der Regel nicht in der Lage, zwischen Enantiomeren von Biomolekülen zu unterscheiden, was für viele Anwendungen in der Biochemie von großer Bedeutung ist.

Der Erfindung liegt daher die Aufgabe zugrunde, eine molekulare Sonde vorzuschlagen, welche die Funktionen der Detektion, der Signaltransduktion sowie der Signalgenerierung in sich vereint und gleichzeitig in einfacher Weise an unterschiedliche Analyte angepasst werden kann.

Diese Aufgabe wird durch eine molekulare Sonde sowie durch ein Material mit den in den unabhängigen Ansprüchen 1 und 14 genannten Merkmalen gelöst. Die erfindungsgemäße molekulare Sonde zum Nachweis eines Analyts umfasst
(a) eine Grundstruktur,
(b) mindestens zwei mittelbar oder unmittelbar an die Grundstruktur gebundene Detektorgruppen, die geeignet sind, gleichzeitig mit dem Analyt in Wechselwirkung zu treten,
(c) mindestens zwei mittelbar oder unmittelbar an die Grundstruktur gebundene Signalgruppen und
(d) mindestens eine signalgebende Konformation der molekularen Sonde, in welcher die Signalgruppen derart miteinander wechselwirken, dass ein detektierbares Signal erzeugt wird, und mindestens eine stumme Konformation, in welcher das detektierbare Signal nicht erzeugt wird.

Sie zeichnet sich dadurch aus, dass die Grundstruktur (GS) 1-Phenyl-1-propin ist, wobei mindestens eine erste Detektorgruppe (D1) und mindestens eine erste Signalgruppe (S1) mittelbar oder unmittelbar über zumindest eine ortho-Position des Phenylrests gebunden sind und mindestens eine zweite Detektorgruppe (D2) und mindestens eine zweite Signalgruppe (S2) mittelbar oder unmittelbar über die C3-Position des Propins gebunden sind.

Die erfindungsgemäße Sonde hat den Vorteil, einerseits alle drei oben beschriebenen Funktionalitäten in einem einzigen Molekül zu vereinen und gleichzeitig eine strenge strukturelle Trennung der drei Funktionen einzuhalten. So sorgt die aus den Detektorgruppen bestehende Detektoreinheit für die Detektion (Bindung) des Analyts, die Grundstruktur mit ihren erlaubten Konformationen für die Signaltransduktion in Form einer Konformationsänderung und die aus den Signalgruppen bestehende Signaleinheit für die Signalgenerierung. Diese modulare Architektur ermöglicht den Austausch einzelner Funktionalitäten und damit eine hochgradig flexible Anpassung der Sonde an das jeweilige Nachweisproblem und die Möglichkeit einer hocheffizienten Optimierung der Signaltransduktion. Beispielsweise kann durch Auswahl geeigneter Detektorgruppen die Spezifität der Sonde für verschiedene Analyte variiert werden. Im Rahmen der hier verwendeten Terminologie wird unter "mittelbarer Bindung" eine Struktur verstanden, bei der eine oder mehrere Signalgruppen nicht direkt (unmittelbar) an die Grundstruktur gebunden vorliegen, sondern über eine oder mehrere Detektorgruppen. Das Gleiche gilt für die Detektorgruppen, die ihrerseits über Signalgruppen gebunden vorliegen können. Ferner wird unter "signalgebende Konformation" eine Konformation verstanden, die ein mit einer geeigneten Methode detektierbares Signal liefert, das von der "stummen Konformation" unterscheidbar ist. Dies kann beispielsweise auch eine Intensitätsänderung (Absorption, Emission, etc.) an einer bestimmten Wellenlänge sein. Somit bedeutet "stumme Konformation" nicht, dass diese keinerlei detektierbares Signal aufweist, sondern dass das detektierbare Signal der signalgebenden Konformation in der stummen Konformation nicht oder nur mit einer vergleichsweise geringeren Intensität vorliegt.

Hinsichtlich der Funktionsweise der erfindungsgemäßen Sonde sind zwei alternative Prinzipien vorgesehen. Gemäß dem ersten Prinzip liegt die mindestens eine signalgebende Konformation vor, wenn die mindestens zwei Detektorgruppen gleichzeitig mit dem Analyt wechselwirken, wobei die mindestens eine stumme Konformation vorliegt, wenn die mindestens zwei Detektorgruppen nicht oder nicht gleichzeitig mit dem Analyt wechselwirken. Nach diesem kooperativen Prinzip erfolgt also unter Anbindung des Analyts an die Detektorgruppen eine Konformationsänderung, welche die Wechselwirkung der Signalgruppen und damit die Signalgenerierung bewirkt. Nach dem zweiten Prinzip liegt die mindestens eine signalgebende Konformation vor, wenn die mindestens zwei Detektorgruppen nicht oder nicht gleichzeitig mit dem Analyt wechselwirken, und die mindestens eine stumme Konformation, wenn die mindestens zwei Detektorgruppen gleichzeitig mit dem Analyt wechselwirken. Nach diesem konkurrierenden Prinzip befinden sich die Signalgruppen in Abwesenheit des Analyts in einer wechselwirkenden und dadurch signalgebenden Position zueinander, die bei der Bindung des Analyts an die Detektorgruppen aufgehoben wird. Infolgedessen kommt es bei Anwesenheit des Analyts zu einer Signallöschung.

Es ist als besonders vorteilhaft anzusehen, dass die erfindungsgemäße molekulare Sonde über eine geringe Zahl von Freiheitsgraden verfügt und ansonsten eine weitgehend starre Struktur aufweist, wobei die signalgebende und stumme Konformation durch eine Konformationsänderung entsprechend einem vorliegenden Freiheitsgrad erreicht werden. Insbesondere beschränkt die Phenyl-1-propin-Struktur die konformativen Freiheitsgrade auf einen einzigen Rotationsfreiheitsgrad um die zu der acetylenischen Dreifachbindung benachbarten C-C-Einfachbindungen.

Für die Wechselwirkung zwischen den mindestens zwei Detektorgruppen und dem Analyt kommen prinzipiell alle bekannten Mechanismen in Frage. Insbesondere kann die Wechselwirkung auf kovalenter, elektrostatischer, Van-der-Waals-, oder Wasserstoffbrückenbindung oder auch einer Kombination von diesen Grundtypen beruhen. Besondere Wechselwirkungen, die im Rahmen der Erfindung bevorzugt eingesetzt werden können, sind etwa Metall-Ligand-, π-Stapelungs-, Protein-Protein-, Peptid-Peptid-, Peptid-Protein-, Protein-Nukleinsäure, Peptid-Nukleinsäure- oder Nukleinsäure-Nukleinsäure-Wechselwirkung oder einer Kombination von diesen. Diese speziellen Wechselwirkungstypen beruhen in der Regel auf einer Kombination der oben genannten Grundtypen.

Die mindestens zwei Detektorgruppen können unterschiedlich oder identisch sein. Sie können insbesondere aus der Gruppe ausgewählt werden umfassend
- Elektronenpaardonatoren, insbesondere Carboxylate; Ethergruppen; Aminogruppen; N-Hetreocyclen, wie Pyridine, Imidazole, und Phenanthroline; Phosphane, Phosphite, Phosphonate und Kronenether;
- Elektronenpaarakzeptoren, insbesondere OH-Gruppen wie Alkohole, Carbonsäuren und Hydroxamsäuren; und NH-Gruppen wie Amide, Amine und N-Heterocyclen;
- Boronsäuren;
- Oligo- und Polypeptide;
- Oligonukleotide;
- Lewissäuren, insbesondere Borane und Boronsäureester; und
- supramolekulare Wirtsmoleküle, insbesondere Cyclodextrine und Cucurbiturile.

Dabei eignen sich Elektronenpaardonatoren besonders zum Nachweis von Kationen; Elektronenpaarakzeptoren zum Nachweis von Anionen; Boronsäuren zum Nachweis von Polyalkoholen, insbesondere Kohlenhydraten; Oligo- und Polypeptide zum Nachweis von Proteinen oder Peptiden, insbesondere von Antikörpern; Oligonukleotide zum Nachweis von Nukleinsäuren, wie RNA oder DNA; Lewissäuren zum Nachweis von Metallkationen und supramolekulare Wirtsmoleküle zum Nachweis kleiner organischer Moleküle.

Hinsichtlich der Signaltransduktion ist bevorzugt vorgesehen, dass in der signalgebenden Konformation die mindestens zwei Signalgruppen eine größere räumliche Nähe zueinander aufweisen als in der stummen Konformation, das heißt, dass mit Bindung des Analyts eine konformative Änderung der Grundstruktur einhergeht, in welcher die Signalgruppen sich entweder zueinander annähern (kooperatives Prinzip) oder voneinander wegbewegen (konkurrierendes Prinzip). Andere Mechanismen der Signaltransduktion, beispielsweise eine Änderung der elektronischen Struktur der molekularen Sonde, sind im Rahmen der vorliegenden Erfindung jedoch auch denkbar.

Die signalgebende Wechselwirkung der mindestens zwei Signalgruppen untereinander beruht vorzugsweise auf Energie-und/oder Ladungstransfer zwischen Grundzustand und angeregten Zuständen der Signalgruppen. Insbesondere kommen hier Mechanismen der Excimerenbildung, des Fluoreszenz-Resonanzenergie-Transfers (FRET) oder der Fluoreszenzlöschung zur Anwendung. Daneben können jedoch auch elektrostatische, Van-der-Waals- oder Wasserstoffbrückenbindungs-Wechselwirkungen oder einer Kombination von diesen auftreten. Die Natur des generierten Signals hängt hauptsächlich von den gewählten Signalgruppen ab. Diese werden vorzugsweise so gewählt, dass das Signal mittels spektroskopischer Methoden, insbesondere NMR-, UV-, UV-VIS-, ESR-, Fluoreszenz- oder FRET-Spektroskopie detektierbar ist. Von diesen Methoden werden die Fluoreszenz- oder FRET-Spektroskopie besonders bevorzugt.

Insbesondere können die mindestens zwei Signalgruppen unterschiedlich oder identisch aus der Gruppe umfassend polyaromatische Kohlenwasserstoffe, wie Pyren- oder Anthrazenderivate, ausgewählt werden. Diese Gruppen eignen sich insbesondere zur fluoreszenzspektroskopischen Detektion der Excimerenemission.

Besonders zur Detektion des Fluoreszenz-Resonanzenergie-Transfers (FRET) eignet sich eine Kombination unterschiedlicher Fluoreszenzfarbstoffe für die Signalgruppen. Diese können insbesondere aus der Gruppe umfassend Fluorescein, Rhodamin, Pyren, Dansyl, Eosin, Diphenyloxazol sowie Derivaten von diesen ausgewählt werden. Bevorzugte Kombinationen sind etwa Fluorescein/Rhodamin, Pyren/Fluorescein, Dansyl/Azobenzol, Fluorescein/Eosin oder Diphenyloxazol/ Fluorescein.

Die Signalgruppen können auch eine Kombination eines Elektronenpaardonators, insbesondere eines tertiären Amins, mit einem Fluoreszenzfarbstoff, insbesondere einem Polyaromaten, sein. Diese Gruppen eignen sich besonders zur Detektion der Fluoreszenzlöschung. Andere Typen geeigneter Signalgruppen sind im Rahmen der Erfindung ebenfalls einsetzbar.

Abhängig von einem vorliegenden Nachweisproblem werden die mindestens zwei Detektorgruppen so gewählt, dass sie geeignet sind, mit einem Molekül, insbesondere einem Peptid, einem Protein, einer Nukleinsäure, einem Kohlenhydrat oder einer niedermolekularen Verbindung, oder mit einem Ion, insbesondere mit einem Metallion, zu wechselwirken.

Die Erfindung betrifft ferner ein Material zum Nachweis eines Analyts, das eine feste Trägerphase, vorzugsweise ein organisches oder anorganisches Polymer, sowie die an einer Oberfläche der Trägerphase immobilisierte molekulare Sonde gemäß der Erfindung umfasst. Die Immobilisierung beruht vorzugsweise auf einer kovalenten Bindung der molekularen Sonde über ihre Grundstruktur.

Die Erfindung betrifft weiter die Verwendung der erfindungsgemäßen molekularen Sonde oder des erfindungsgemäßen Materials zum Nachweis eines Analyts. Zu diesem Zweck wird die molekulare Sonde bzw. das Material mit dem nachzuweisenden Analyt in Kontakt gebracht und mittels eines geeigneten spektroskopischen Verfahrens ein detektierbares Signal vor, während und/oder nach erfolgter Reaktion zwischen der Sonde und dem Analyt gemessen. Der qualitative Nachweis des Analyts ergibt sich dann aus dem Vorhandensein (beim kooperativen Prinzip) beziehungsweise der Abwesenheit des Signals (beim konkurrierenden Prinzip). Eine quantitative Auswertung kann über eine Bestimmung der Signalintensität erfolgen.

Weitere bevorzugte Ausgestaltungen der Erfindung ergeben sich aus den übrigen, in den Unteransprüchen genannten Merkmalen.

Die Erfindung wird nachfolgend in Ausführungsbeispielen anhand der zugehörigen Zeichnungen näher erläutert. Es zeigen:
- Figur 1A: eine allgemeine Struktur einer molekularen Sonde nach dem kooperativen Funktionsprinzip;
- Figur 1B: eine erste allgemeine Struktur der molekularen Sonde nach dem konkurrierenden Funktionsprinzip;
- Figur 1C: eine zweite allgemeine Struktur der molekularen Sonde nach dem konkurrierenden Funktionsprinzip;
- Figur 2A: eine spezielle Struktur der erfindungsgemäßen molekularen Sonde nach dem kooperativen Funktionsprinzip und deren Reaktionsmechanismus;
- Figur 2B: eine spezielle Struktur der erfindungsgemäßen molekularen Sonde nach dem konkurrierenden Funktionsprinzip und deren Reaktionsmechanismus;
- Figur 3: eine bevorzugte Ausgestaltung einer ersten molekularen Sonde;
- Figur 4: eine bevorzugte Ausgestaltung einer zweiten molekularen Sonde; und
- Figur 5: ein Beispiel für einen bevorzugten Syntheseweg der molekularen Sonde gemäß Figur 2A und 2B.

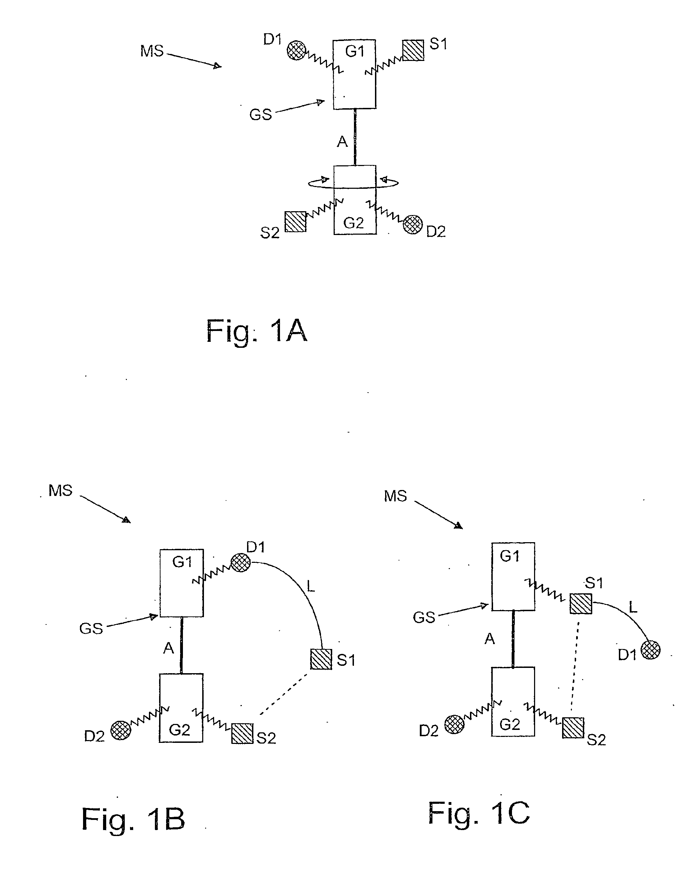

Figur 1A zeigt eine allgemeine Struktur einer insgesamt mit MS bezeichneten molekularen Sonde, deren Funktionsweise auf einem kooperativen Prinzip beruht. Die molekulare Sonde MS umfasst eine Grundstruktur GS, die ihrerseits aus einer molekularen Achse A, einer an einem ersten Achsenende der Achse A gebundenen Gruppe G1 und einer an einem zweiten Achsenende gebundenen Gruppe G2 besteht. Die molekulare Achse A weist eine starre lineare Struktur auf und erlaubt lediglich die durch den Doppelpfeil angedeutete Rotation der Gruppen G1 und/oder G2.

Die molekulare Sonde MS umfasst ferner eine Detektoreinheit, die mindestens eine erste Detektorgruppe D1 und mindestens eine zweite Detektorgruppe D2 umfasst, wobei jeweils mindestens eine Detektorgruppe D1, D2 an eine Gruppe G1 beziehungsweise G2 gebunden vorliegt. Die molekulare Sonde MS umfasst ferner eine aus mindestens einer ersten Signalgruppe S1 und mindestens einer zweiten Signalgruppe S2 bestehenden Signaleinheit, wobei auch hier jeweils mindestens eine Signalgruppe S1, S2 an eine Gruppe G1 beziehungsweise G2 gebunden ist.

Die mindestens zwei Detektorgruppen D1, D2 sind in der Lage, gleichzeitig mit einem nachzuweisenden Analyt spezifisch zu wechselwirken. Kommt es zu einer auf beliebigen Wechselwirkungseffekten beruhenden Bindung eines Analyts durch beide Detektorgruppen D1 und D2, so wird hierdurch eine Rotation der Grundstruktur verursacht, wodurch die Signalgruppen S1 und S2 zu einer räumlichen Annäherung zueinander gelangen. Infolge der resultierenden Wechselwirkung der Signalgruppen S1, S2 wird ein detektierbares Signal, beispielsweise ein Fluoreszenzsignal, erzeugt, das fluoreszenzspektrometrisch erfasst werden kann (signalgebende Konformation).

Figur 1B zeigt eine allgemeine Struktur einer molekularen Sonde MS, die nach einem konkurrierenden Prinzip arbeitet. Gleiche Elemente sind mit den gleichen Bezugszeichen wie in Figur 1A bezeichnet. In Abweichung von der nach dem kooperativen Prinzip funktionierenden Sonde nach Figur 1A ist hier die Signalgruppe S1 nicht direkt an die erste Gruppe G1 gebunden, sondern mittelbar über eine ihrerseits an die erste Detektorgruppe D1 gebundene Linkergruppe L, die beispielsweise eine alkylische Struktur sein kann.

Die konkurrierend funktionierende Sonde MS nach Figur 1B liegt - wie dargestellt - in Abwesenheit eines nachzuweisenden Analyts in einer signalgebenden Konformation vor. In dieser Konformation kommt es zur Wechselwirkung der Signalgruppen S1 und S2 und damit zu einer Signalerzeugung. In Gegenwart des Analyts erfolgt eine Bindung desselben an die Detektorgruppen D1 und D2, wodurch eine Rotation der Grundstruktur GS und eine Aufhebung der Wechselwirkung der Signalgruppen S1 und S2 hervorgerufen wird. Dies setzt voraus, dass die Wechselwirkungskräfte zwischen Analyt und Detektorgruppen größer sind als die der Signalgruppen untereinander. Infolge der Bindung des Analyts an die Detektorgruppen D1 und D2 kommt es zu einer Signallöschung (stumme Konformation).

Eine zweite Variante einer nach dem konkurrierenden Prinzip funktionierenden Sonde ist in Figur 1C dargestellt. Im Unterschied zu der Sonde nach Figur 1B ist hier die erste Detektorgruppe D1 über die ihrerseits an die erste Gruppe G1 gebundene erste Signalgruppe S1 mit der Grundstruktur GS verbunden. Wie bei der Sonde nach Figur 1B liegt auch hier in Abwesenheit des Analyts die signalgebende Konformation vor, während unter Bindung desselben durch die Detektorgruppen D1 und D2 die Signalerzeugung aufgehoben wird.

In allen Grundstrukturen der molekularen Sonde nach den Figuren 1A bis 1C können auch mehr als eine Detektor-und/oder Signalgruppe an den Gruppen G1, G2 gebunden vorliegen. Es kann insbesondere vorteilhaft sein, jeweils zwei Detektorgruppen an jeder Gruppe G1 und G2 vorzusehen. Ferner kann die Linkergruppe L nach Figur 1B und 1C auch durch eine einfache Bindung zwischen Detektor- und Signalgruppe ersetzt sein.

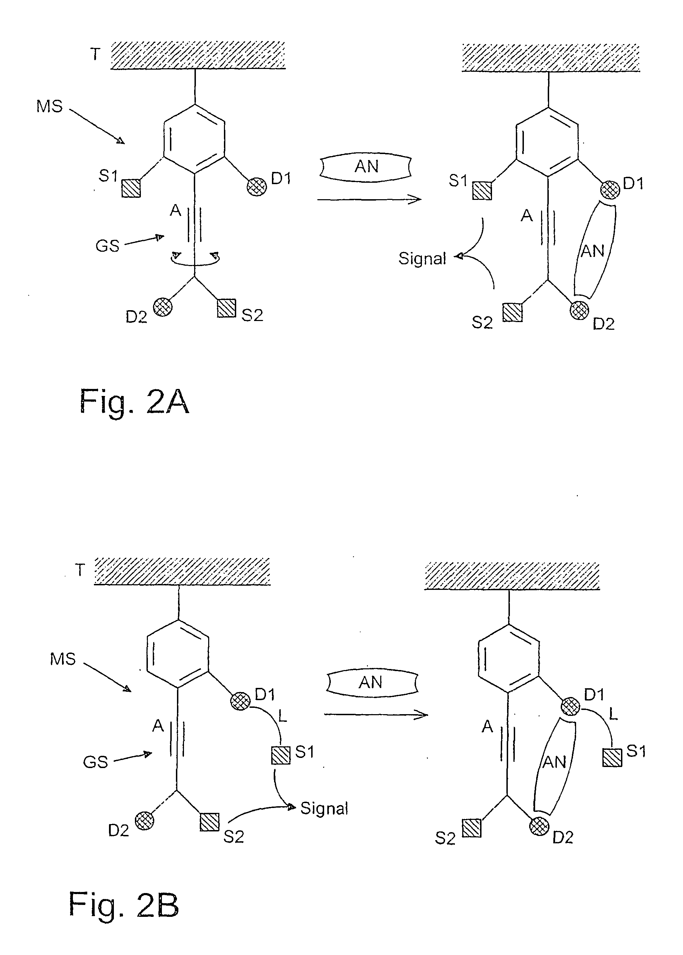

Eine erfindungsgemäße Ausgestaltung der nach dem kooperativen Funktionsprinzip arbeitenden Sonde sowie deren Reaktionsmechanismus ist in Figur 2A dargestellt. Hier handelt es sich bei der Grundstruktur um 1-Phenyl-1-propin. Die erste Detektorgruppe D1 und die erste Signalgruppe S1 liegen jeweils in ortho-Position zu der Propingruppe als unmittelbare Substituenten des Phenylrests vor. Auf der anderen Seite sind die zweite Detektorgruppe D2 und die zweite Signalgruppe S2 unmittelbar an der C3-Position des Propins gebunden.

Die molekulare Sonde MS liegt immobilisiert auf einer Oberfläche einer festen Trägerphase T vor. Insbesondere ist sie über eine kovalente Bindung des Phenylrests in para-Position an die Trägerphase T gebunden. Durch die Immobilisierung der Sonde an der vorzugsweise polymeren Trägerphase T lässt sich eine einfachere Handhabung sowie eine leichte Regeneration des Materials erzielen. Bei der Trägerphase T kann es sich etwa um ein organisches Polymer oder ein anorganisches Polymer, wie Glas oder Keramik, handeln. Um eine Anbindung der Sonde MS an die Trägerphase T zu ermöglichen, kann eine vorherige Funktionalisierung der Trägerphase T notwendig oder hilfreich sein. Entsprechende Verfahren, wie beispielsweise Silanisierung von Glas, sind dem Fachmann hinreichend bekannt.

In Gegenwart des Analyts AN kommt es nach Figur 2A zu einer Bindung gleichzeitig an beide Detektorgruppen D1 und D2. Infolge der hierfür notwendigen Rotation um die C2/C3-Einfachbindung des Propins erlangen die Signalgruppen S1 und S2 eine räumliche Nähe zueinander, die eine Wechselwirkung und Signalgenerierung bewirkt. In der Darstellung entspricht die auf der linken Seite gezeigte freie Konformation der stummen Konformation und die auf der rechten Seite gezeigte Konformation der signalgebenden Konformation.

Figur 2B zeigt eine bevorzugte Struktur einer nach dem konkurrierenden Prinzip funktionierenden erfindungsgemäßen molekularen Sonde. In Abweichung zu Figur 2A ist die erste Signalgruppe S1 nicht direkt an den Phenylrest gebunden, sondern mittelbar über die ihrerseits an die erste Detektorgruppe D1 gebundene Linkergruppe L. In Abwesenheit des Analyts AN liegt die Sonde in der signalgebenden Konformation vor, in der die Signalgruppen S1 und S2 miteinander wechselwirken und ein Signal erzeugen (linke Seite). Sobald das Analyt AN gleichzeitig an beide Detektorgruppen D1 und D2 bindet, erfolgt eine Rotation um die C2/C3-Einfachbindung des Propins und damit Signallöschung. Diese stumme Konformation ist auf der rechten Seite der Figur 2B gezeigt.

### Beispiel 1:

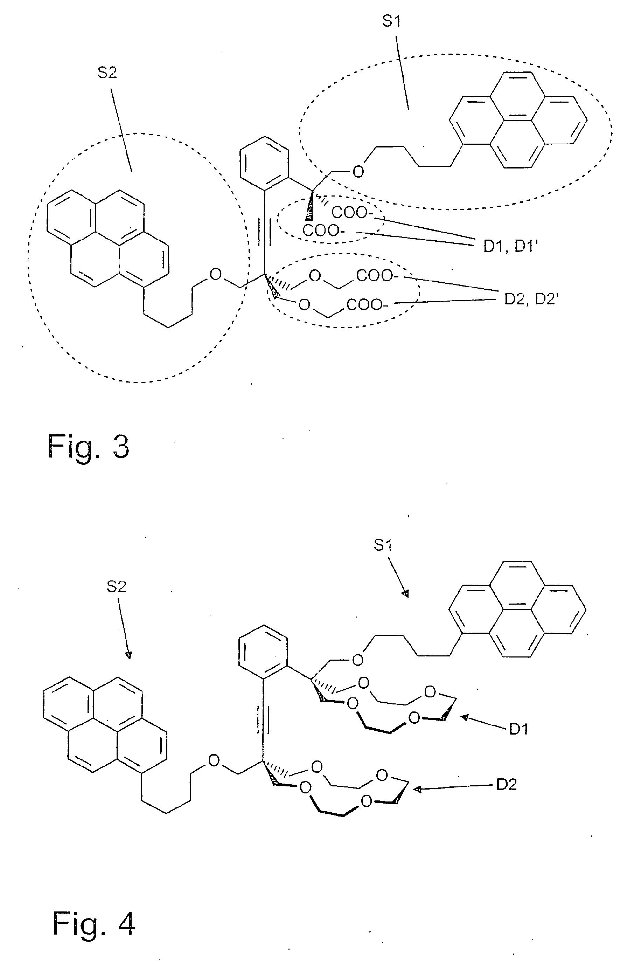

Ein konkretes Beispiel für eine molekulare Sonde MS, die etwa zum Nachweis von Metallionen geeignet ist, zeigt Figur 3. Die Sonde weist als Grundstruktur 1-Phenyl-1-propin auf. An der Methylgruppe des Phenyls sowie an der C3-Position des Propins ist jeweils eine 4-(1-Pyrenyl)-butoxymethyl-Gruppe als erste beziehungsweise zweite Signalgruppe S1 und S2 gebunden. Ferner sind an der Methylgruppe des Phenyls zwei Carboxylatgruppen' als erste Detektorgruppen D1 und D1' gebunden. Auf der anderen Seite sind an der C3-Position des Propins zwei Carboxymethoxymethyl-Gruppen als zweite Detektorgruppen D2 und D2' gebunden.

Die in Figur 3 gezeigte Struktur entspricht der stummen Konformation, in der ein (nicht dargestelltes) Metallion durch die vier Carboxylgruppen der Detektorgruppen D1, D1', D2 und D2' sowie der Ethergruppen der Detektorgruppen D2 und D2' durch elektrostatische Wechselwirkungen komplexiert wird. In dieser Konformation wechselwirken die beiden Signalgruppen S1 und S2 nicht miteinander. In Abwesenheit des Metallions treten die Signalgruppen S1 und S2 unter Excimerenbildung in Wechselwirkung, wobei eine Assoziatbildung zwischen einer Pyrengruppe im Grundzustand und einer in einem elektronisch angeregten Zustand befindlichen Pyrengruppe stattfindet. Die so entstehende Excimerenemission kann fluoreszenzspektroskopisch erfasst werden.

### Beispiel 2:

Figur 4 zeigt ein weiteres konkretes Beispiel für eine molekulare Sonde MS. In Abweichung zu der Struktur nach Figur 3 sind hier zwei Kronenetherstrukturen des Typs 13-Krone-4 als erste und zweite Detektorgruppe D1 und D2 über die Methylgruppe des Phenyls beziehungsweise über die C3-Position des Propins mit der Grundstruktur verbunden. Diese Detektorgruppen D1, D2 sind geeignet, ein Kation, insbesondere ein Metallkation, zu binden.

### Synthesevorschrift:

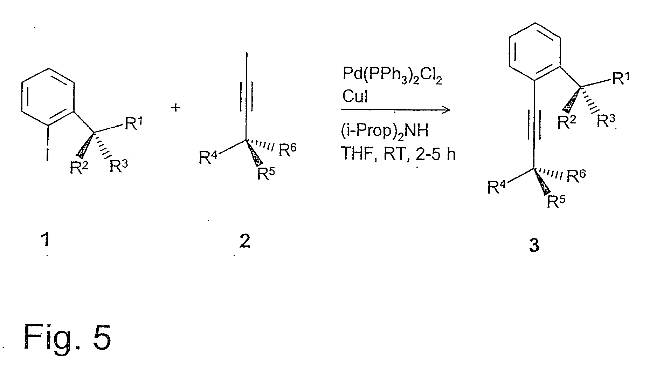

Erfindungsgemäße molekulare Sonden mit einer Grundstruktur bestehend aus 1-Phenyl-1-propin gemäß Figuren 2A oder 2B, insbesondere gemäß Beispiel 1 oder 2, können nach der allgemeinen Gleichung gemäß Figur 4 synthetisiert werden. In Figur 5 bedeuten die Reste R¹ bis R⁶ entsprechende Detektor- oder Signalgruppen oder H mit den Vorgaben, dass die Gruppen R¹, R² und R³ eine oder zwei Detektorgruppen und eine oder zwei Signalgruppen umfassen und auch die Gruppen R⁴, R⁵ und R⁶ eine oder zwei Detektorgruppen und eine oder zwei Signalgruppen umfassen. Die Synthese geht von der Propingruppe 2 aus, die an ihrer C3-Position mit den Resten R¹, R² und R³ substituiert ist, sowie von dem Iodaromaten 1, insbesondere 2-Iodtoluol, das an seiner Methylgruppe mit den Resten R⁴, R⁵ und R⁶ substituiert ist.

2,84 mmol des Propins 2, 2,84 mmol des Iodaromaten 1, 0,48 ml Diisopropylamin (1,2 Äqu.), 54 mg Kupfer-I-Iodid (10 mol-%) und 80 mg Bis-(triphenylphosphan)-palladium-II-chlorid (4 mol-%) werden in 20 ml Tetrahydrofuran bei Raumtemperatur und Schutzgas (N₂) zusammengegeben. Die Reaktionslösung verfärbt sich von orange über rot nach dunkelbraun. Der Fortgang der Reaktion wird am Vorhandensein des Iodaromaten 1 mittels Dünnschichtchromatographie ermessen. Nach ca. 2 h ist die Reaktion beendet. Es werden 100 ml Diethylether zugegeben, nacheinander mit wässriger Weinsäurelösung (10%ig) und Wasser gewaschen; die etherische Lösung mit Magnesiumsulfat getrocknet und am Rotationsverdampfer eingeengt. Der Rückstand wird mittels Flash-Chromatographie an Kieselgel gereinigt (Laufmittel Dichlormethan). Die Ausbeuten am Produkt 3 (molekulare Sonde) betrugen 85 bis 95 %.

### Bezugszeichenliste

- A: molekulare Achse
- AN: Analyt
- D1: erste Detektorgruppe
- D2: zweite Detektorgruppe
- G1: erste Gruppe
- G2: zweite Gruppe
- GS: Grundstruktur
- L: Linkergruppe
- MS: molekulare Sonde
- S1: erste Signalgruppe
- S2: zweite Signalgruppe
- T: Trägerphase

## Patentansprüche

1. Molekulare Sonde (MS) zum Nachweis eines Analyts (AN), umfassend
(a) eine Grundstruktur (GS),
(b) mindestens zwei mittelbar oder unmittelbar an die Grundstruktur (GS) gebundene Detektorgruppen (D1, D2), die geeignet sind, gleichzeitig mit dem Analyt (AN) in Wechselwirkung zu treten,
(c) mindestens zwei mittelbar oder unmittelbar an die Grundstruktur (GS) gebundene Signalgruppen (S1, S2), und
(d) mindestens eine signalgebende Konformation der molekularen Sonde (MS), in welcher die Signalgruppen (S1, S2) derart miteinander wechselwirken, dass ein detektierbares Signal erzeugt wird, und mindestens eine stumme Konformation, in welcher das detektierbare Signal nicht erzeugt wird,
**dadurch gekennzeichnet, dass**
die Grundstruktur (GS) 1-Phenyl-1-propin ist, wobei mindestens eine erste Detektorgruppe (D1) und mindestens eine erste Signalgruppe (S1) mittelbar oder unmittelbar über zumindest eine ortho-Position des Phenylrests gebunden sind und mindestens eine zweite Detektorgruppe (D2) und mindestens eine zweite Signalgruppe (S2) mittelbar oder unmittelbar über die C3-Position des Propins gebunden sind.

2. Molekulare Sonde (MS) nach Anspruch 1, **dadurch gekennzeichnet, dass** die mindestens eine signalgebende Konformation vorliegt, wenn die mindestens zwei Detektorgruppen (D1, D2) gleichzeitig mit dem Analyt (AN) wechselwirken, und die mindestens eine stumme Konformation vorliegt, wenn die mindestens zwei Detektorgruppen (D1, D2) nicht oder nicht gleichzeitig mit dem Analyt (AN) wechselwirken.

3. Molekulare Sonde (MS) nach Anspruch 1, **dadurch gekennzeichnet, dass** die mindestens eine signalgebende Konformation vorliegt, wenn die mindestens zwei Detektorgruppen (D1, D2) nicht oder nicht gleichzeitig mit dem Analyt (AN) wechselwirken, und die mindestens eine stumme Konformation vorliegt, wenn die mindestens zwei Detektorgruppen (D1, D2) gleichzeitig mit dem Analyt (AN) wechselwirken.

4. Molekulare Sonde (MS) nach einem der vorhergehenden Ansprüche, **dadurch gekennzeichnet, dass** die Wechselwirkung zwischen den mindestens zwei Detektorgruppen (D1, D2) und dem Analyt (AN) auf kovalenter Bindung, elektrostatischer Bindung, Van-der-Waals-Bindung oder Wasserstoffbrückenbindung oder einer Kombination von diesen beruht.

5. Molekulare Sonde (MS) nach Anspruch 4, **dadurch gekennzeichnet, dass** die Wechselwirkung zwischen den mindestens zwei Detektorgruppen (D1, D2) und dem Analyt (AN) auf Metall-Ligand-, π-Stapelungs-, Protein-Protein-, Peptid-Peptid-, Peptid-Protein-, Protein-Nukleinsäure, Peptid-Nukleinsäure- oder Nukleinsäure-Nukleinsäure-Wechselwirkung oder einer Kombination von diesen beruht.

6. Molekulare Sonde (MS) nach einem der vorhergehenden Ansprüche, **dadurch gekennzeichnet, dass** die mindestens zwei Detektorgruppen (D1, D2) unterschiedlich oder identisch sind und ausgewählt sind aus der Gruppe umfassend
- Elektronenpaardonatoren, insbesondere Carboxylate; Ethergruppen; Aminogruppen; N-Hetreocyclen, wie Pyridine, Imidazole, und Phenanthroline; Phosphane, Phosphite, Phosphonate und Kronenether;
- Elektronenpaarakzeptoren, insbesondere OH-Gruppen, wie Alkohole, Carbonsäuren und Hydroxamsäuren; und NH-Gruppen, wie Amide, Amine und N-Heterocyclen;
- Boronsäuren;
- Oligo- und Polypeptide;
- Oligonukleotide;
- Lewissäuren, insbesondere Borane und Boronsäureester; und
- supramolekulare Wirtsmoleküle, insbesondere Cyclodextrine und Cucurbiturile.

7. Molekulare Sonde (MS) nach einem der vorhergehenden Ansprüche, **dadurch gekennzeichnet, dass** in der signalgebenden Konformation die mindestens zwei Signalgruppen (S1, S2) eine größere räumliche Nähe zueinander aufweisen als in der stummen Konformation.

8. Molekulare Sonde (MS) nach einem der vorhergehenden Ansprüche, **dadurch gekennzeichnet, dass** die Wechselwirkung der mindestens zwei Signalgruppen (S1, S2) untereinander auf Energie- und/oder Ladungstransfer zwischen Grundzustand und angeregtem Zustand, insbesondere Excimerenbildung, FRET und/oder Fluoreszenzlöschung; auf elektrostatischer Bindung, Van-der-Waals-Bindung oder Wasserstoffbrückenbindung oder einer Kombination von diesen beruht.

9. Molekulare Sonde (MS) nach einem der vorhergehenden Ansprüche, **dadurch gekennzeichnet, dass** das Signal mittels mindestens einer der folgenden spektroskopischen Methoden detektierbar ist: NMR-, UV-, UV-VIS-, ESR-, Fluoreszenz-, Fluoreszenz-Resonanzenergie-Transfer-Spektroskopie.

10. Molekulare Sonde (MS) nach einem der Ansprüche 1 bis 9, **dadurch gekennzeichnet, dass** die mindestens zwei Signalgruppen (S1, S2) unterschiedlich oder identisch sind und ausgewählt sind aus der Gruppe umfassend polyaromatische Kohlenwasserstoffe, insbesondere Pyren- und Anthrazenderivate.

11. Molekulare Sonde (MS) nach einem der Ansprüche 1 bis 9, **dadurch gekennzeichnet, dass** die mindestens zwei Signalgruppen (S1, S2) eine Kombination unterschiedlicher Fluoreszenzfarbstoffe sind, insbesondere ausgewählt sind aus der Gruppe umfassend Fluorescein, Rhodamin, Pyren, Dansyl, Eosin und Diphenyloxazol.

12. Molekulare Sonde (MS) nach einem der Ansprüche 1 bis 9, **dadurch gekennzeichnet, dass** die mindestens zwei Signalgruppen (S1, S2) eine Kombination eines Elektronenpaardonators, insbesondere eines tertiären Amins, mit einem Fluoreszenzfarbstoff, insbesondere einem Polyaromat, sind.

13. Molekulare Sonde (MS) nach einem der vorhergehenden Ansprüche, **dadurch gekennzeichnet, dass** die mindestens zwei Detektorgruppen (D1, D2) geeignet sind, mit einem Analyt (AN) zu wechselwirken, das ein Molekül, insbesondere ein Peptid, ein Protein, eine Nukleinsäure, ein Kohlenhydrat oder eine niedermolekulare Verbindung, oder ein Ion, insbesondere ein Metallion, ist.

14. Material zum Nachweis eines Analyts (AN) umfassend eine feste Trägerphase (T) und eine an einer Oberfläche der Trägerphase (T) immobilisierten molekularen Sonde (MS) nach einem der Ansprüche 1 bis 13.

15. Material nach Anspruch 14, **dadurch gekennzeichnet, dass** die molekulare Sonde (MS) über ihre Grundstruktur (GS) kovalent an die Trägerphase (T) gebunden ist.

16. Material nach Anspruch 14 oder 15, **dadurch gekennzeichnet, dass** die Trägerphase (T) ein organisches oder anorganisches Polymer ist.

17. Verwendung einer molekularen Sonde (MS) nach einem der Ansprüche 1 bis 13 oder eines Materials nach einem der Ansprüche 14 bis 16 zum Nachweis eines Analyts (AN), wobei die molekulare Sonde (MS) beziehungsweise das Material mit dem nachzuweisenden Analyt (AN) in Kontakt gebracht wird und mittels eines spektroskopischen Verfahrens ein detektierbares Signal vor, während und/oder nach erfolgter Reaktion zwischen molekularer Sonde (MS) und Analyt (AN) gemessen wird.

## Claims

1. A molecular probe (MS) for the detection of an analyte (AN), comprising
(a) a base structure (GS),
(b) at least two detector groups (D1, D2) bound indirectly or directly to the base structure (GS) and suited to undergo simultaneous interaction with the analyte (AN),
(c) at least two signal groups (S1, S2) bound indirectly or directly to the base structure (GS), and
(d) at least one signaling conformation of the molecular probe (MS), in which the signal groups (S1, S2) interact in such a way that a detectable signal is generated, and at least one silent conformation in which said detectable signal is not generated,
**characterized in that**
the base structure (GS) is 1-phenyl-1-propine, at least one first detector group (D1) and at least one first signal group (S1) being indirectly or directly bound via at least one ortho position of the phenyl residue and at least one second detector group (D2) and at least one second signal group (S2) being indirectly or directly bound via the C3 position of the propine.

2. The molecular probe (MS) according to claim 1, **characterized in that** said at least one signaling conformation is present if said at least two detector groups (D1, D2) simultaneously interact with the analyte (AN), and said at least one silent conformation is present if said at least two detector groups (D1, D2) do not interact or do not simultaneously interact with the analyte (AN).

3. The molecular probe (MS) according to claim 1, **characterized in that** said at least one signaling conformation is present if said at least two detector groups (D1, D2) do not interact or do not simultaneously interact with the analyte (AN), and said at least one silent conformation is present if said at least two detector groups (D1, D2) simultaneously interact with the analyte (AN).

4. The molecular probe (MS) according to any of the preceding claims, **characterized in that** the interaction between said at least two detector groups (D1, D2) and the analyte (AN) is based on covalent binding, electrostatic binding, Van-der-Waals binding or hydrogen bridges or a combination thereof.

5. The molecular probe (MS) according to claim 4, **characterized in that** the interaction between said at least two detector groups (D1, D2) and the analyte (AN) is based on metal-ligand, π-stacking, protein-protein, peptide-peptide, peptide-protein, protein-nucleic acid, peptide-nucleic acid or nucleic acid-nucleic acid interaction or a combination thereof.

6. The molecular probe (MS) according to any of the preceding claims, **characterized in that** said at least two detector groups (D1, D2) are different or identical and selected from the group comprising
- electron pair donors, particularly carboxylates; ether groups; amino groups; N-heterocycles such as pyridines, imidazoles and phenanthrolines; phosphanes, phosphites, phosphonates, and crown ethers;
- electron pair acceptors, particularly OH groups, such as alcohols, carboxylic acids and hydroxamic acids; and NH groups, such as amides, amines and N-heterocycles;
- boronic acids;
- oligo- and polypeptides;
- oligonucleotides;
- Lewis acids, particularly boranes and boronic esters; and
- supramolecular host molecules, particularly cyclodextrins and cucurbiturils.

7. The molecular probe (MS) according to any of the preceding claims, **characterized in that** said at least two signal groups (S1, S2) in the signaling conformation are in closer spatial proximity to each other compared to the silent conformation.

8. The molecular probe (MS) according to any of the preceding claims, **characterized in that** the interaction between said at least two signal groups (S1, S2) is based on energy and/or charge transfer between ground state and excited state, particularly formation of excimers, FRET and/or fluorescence quenching; electrostatic binding, Van-der-Waals binding or hydrogen bridges or a combination thereof.

9. The molecular probe (MS) according to any of the preceding claims, **characterized in that** the signal is detectable by means of at least one of the following spectroscopic methods: NMR, UV, UV-VIS, ESR, fluorescence, fluorescence resonance energy transfer spectroscopy.

10. The molecular probe (MS) according to any of claims 1 to 9, **characterized in that** said at least two signal groups (S1, S2) are different or identical and selected from the group comprising polyaromatic hydrocarbons, particularly pyrene and anthracene derivatives.

11. The molecular probe (MS) according to any of claims 1 to 9, **characterized in that** said at least two signal groups (S1, S2) are a combination of different fluorescent dyes, particularly those selected from the group of fluorescein, rhodamine, pyrene, dansyl, eosin and diphenyloxazole.

12. The molecular probe (MS) according to any of claims 1 to 9, **characterized in that** said at least two signal groups (S1, S2) are a combination of an electron pair donor, particularly a tertiary amine, and a fluorescent dye, particularly a polyaromatic substance.

13. The molecular probe (MS) according to any of the preceding claims, **characterized in that** said at least two detector groups (D1, D2) are suited to interact with an analyte (AN) which is a molecule, particularly a peptide, a protein, a nucleic acid, a carbohydrate or a low-molecular weight compound, or an ion, particularly a metal ion.

14. A material for the detection of an analyte (AN), comprising a solid carrier phase (T) and a molecular probe (MS) according to any of claims 1 to 13 immobilized on a surface of the carrier phase (T).

15. The material according to claim 14, **characterized in that** the molecular probe (MS) is covalently bound via its base structure (GS) to the carrier phase (T).

16. The material according to claim 14 or 15, **characterized in that** the carrier phase (T) is an organic or inorganic polymer.

17. Use of a molecular probe (MS) according to any of claims 1 to 13 or a material according to any of claims 14 to 16 for the detection of an analyte (AN), wherein the molecular probe (MS) or the material is contacted with the analyte (AN) to be detected and, using a spectroscopic method, a detectable signal is measured prior to, during and/or subsequent to the reaction proceeding between molecular probe (MS) and analyte (AN).

## Revendications

1. Sonde moléculaire (MS) de détection d'un analyte (AN), comprenant
(a) une structure primaire (GS),
(b) au moins deux groupes détecteurs (D1, D2) liés indirectement ou directement à la structure primaire (GS), qui sont appropriés pour entrer en interaction simultanément avec l'analyte (AN),
(c) au moins deux groupes signaux (S1, S2) liés indirectement ou directement à la structure primaire (GS), et
(d) au moins une conformation donnant un signal de la sonde moléculaire (MS), dans laquelle les groupes signaux (S1, S2) interagissent l'un avec l'autre de telle sorte qu'un signal détectable est généré, et au moins une conformation muette, dans laquelle le signal détectable n'est pas généré,
**caractérisée en ce que**
la structure primaire (GS) est du 1-phényl-1-propine, moyennant quoi au moins un premier groupe détecteur (D1) et au moins un premier groupe signal (S1) sont liés indirectement ou directement par l'intermédiaire d'au moins une position ortho du résidu phényl et au moins un second groupe détecteur (D2) et au moins un second groupe signal (S2) sont liés indirectement ou directement par l'intermédiaire de la position C3 du propine.

2. Sonde moléculaire (MS) selon la revendication 1, **caractérisée en ce que** la au moins une conformation donnant un signal existe si les au moins deux groupes détecteurs (D1, D2) interagissent en même temps avec l'analyte (AN) et la au moins une conformation muette existe si les au moins deux groupes détecteurs (D1, D2) n'interagissent pas ou pas simultanément avec l'analyte (AN).

3. Sonde moléculaire (MS) selon la revendication 1, **caractérisée en ce que** la au moins une conformation donnant un signal existe si les au moins deux groupes détecteurs (D1, D2) n'interagissent pas ou pas simultanément avec l'analyte (AN) et la au moins une conformation muette existe si les au moins deux groupes détecteurs (D1, D2) interagissent simultanément avec l'analyte (AN).

4. Sonde moléculaire (MS) selon l'une des revendications précédentes, **caractérisée en ce que** l'interaction entre les au moins deux groupes détecteurs (D1, D2) et l'analyte (AN) repose sur la liaison covalente, la liaison électrostatique, la liaison de Van der Waals ou la liaison de pont d'hydrogène ou une combinaison de celles-ci.

5. Sonde moléculaire (MS) selon la revendication 4, **caractérisée en ce que** l'interaction entre les au moins deux groupes détecteurs (D1, D2) et l'analyte (AN) repose sur l'interaction métal-ligand, l'interaction d'empilement π, l'interaction protéine-protéine, l'interaction peptide-peptide, l'interaction peptide-protéine, l'interaction protéine-acide nucléique, l'interaction peptide-acide nucléique ou l'interaction acide nucléique-acide nucléique ou une combinaison de celles-ci.

6. Sonde moléculaire (MS) selon l'une des revendications précédentes, **caractérisée en ce que** les au moins deux groupes détecteurs (D1, D2) sont différents ou identiques et qu'ils sont sélectionnés parmi le groupe comprenant :
- les donneurs de paire d'électrons, notamment les carboxylates; les groupes éther; les groupes amino; les N-hétérocycles, comme les pyridines, les imidazoles et les phénanthrolines; les phosphanes, les phosphites, les phosphonates et les éthers-couronnes;
- les accepteurs de paire d'électrons, notamment les groupes OH, comme les alcools, les acides carboxyliques et les acides hydroxamiques; et les groupes NH, comme les amides, les amines et les N-hétérocycles;
- les acides boroniques;
- les oligo- et polypeptides;
- les oligonucléotides;
- les acides de Lewis, notamment les boranes et les esters d'acide borique; et
- les molécules hôtes supramoléculaires, notamment les cyclodextrines et les cucurbituriles.

7. Sonde moléculaire (MS) selon l'une des revendications précédentes, **caractérisée en ce que** les au moins deux groupes signaux (S1, S2) dans la conformation donnant un signal présentent une plus grande proximité spatiale que dans la conformation muette.

8. Sonde moléculaire (MS) selon l'une des revendications précédentes, **caractérisée en ce que** l'interaction des au moins deux groupes signaux (S1, S2) entre eux repose sur un transfert d'énergie et/ou de charge entre l'état fondamental et l'état excité, notamment la formation d'excimères, le FRET et/ou le déclin de fluorescence; sur la liaison électrostatique, la liaison de Van der Waals ou la liaison par pont hydrogène ou une combinaison de celles-ci.

9. Sonde moléculaire (MS) selon l'une des revendications précédentes, **caractérisée en ce que** le signal est détectable au moyen d'au moins une des méthodes spectroscopique suivantes : spectroscopie RMN, spectroscopie UV, UV-VIS, RPE, spectroscopie de fluorescence, spectroscopie de transfert d'énergie de résonance de fluorescence.

10. Sonde moléculaire (MS) selon l'une des revendications 1 à 9, **caractérisée en ce que** les au moins deux groupes signaux (S1, S2) sont différents ou identiques et sont sélectionnés parmi le groupe comprenant les hydrocarbures polyaromatiques, notamment les dérivés de pyrène et d'anthracène.

11. Sonde moléculaire (MS) selon l'une des revendications 1 à 9, **caractérisée en ce que** les au moins deux groupes signaux (S1, S2) sont une combinaison de différents colorants de fluorescence, et sont sélectionnés notamment parmi le groupe comprenant la fluorescéine, la rhodamine, le pyrène, le dansyle, l'éosine et le diphényloxazole.

12. Sonde moléculaire (MS) selon l'une des revendications 1 à 9, **caractérisée en ce que** les au moins deux groupes signaux (S1, S2) sont une combinaison d'un donneur de paire d'électrons, notamment d'un amine tertiaire, et d'un colorant de fluorescence, notamment un polyaromatique.

13. Sonde moléculaire (MS) selon l'une des revendications précédentes, **caractérisée en ce que** les au moins deux groupes détecteurs (D1, D2) conviennent pour interagir avec un analyte (AN), qui est une molécule, notamment un peptide, une protéine, un acide nucléique, un hydrate de carbone ou un composé à bas poids moléculaire, ou un ion, notamment un ion de métal.

14. Matériau de détection d'un analyte (AN) comprenant une phase de support solide (T) et une sonde moléculaire (MS) selon l'une des revendications 1 à 13 fixée sur une surface de la phase de support (T).

15. Matériau selon la revendication 14, **caractérisé en ce que** la sonde moléculaire (MS) est liée par sa structure primaire (GS) de manière covalente à la phase de support (T).

16. Matériau selon la revendication 14 ou 15, **caractérisé en ce que** la phase de support (T) est un polymère organique ou inorganique.

17. Utilisation d'une sonde moléculaire (MS) selon l'une des revendications 1 à 13 ou d'un matériau selon l'une des revendications 14 à 16 pour détecter un analyte (AN), moyennant quoi on met en contact la sonde moléculaire (MS) ou le matériau avec l'analyte (AN) à détecter et, à l'aide d'une méthode spectroscopique, on mesure un signal détectable avant, pendant et/ou après la réaction ayant lieu entre la sonde moléculaire (MS) et l'analyte (AN).
